# EUROPEAN PATENT SPECIFICATION

(11) **EP 0 399 523 B1**
(45) Date of publication and mention of the grant of the patent: **05.04.1995**
(21) Application number: 90109868.1
(22) Date of filing: 23.05.1990
(51) Int. Cl.: A45D 27/04, A45D 34/04

(54) **Pocket-sized bottle for distributing a shaving soap emulsion over the skin, and the relative soap emulsion**
Flasche von Taschenformat zum Verteilen einer Rasier-Seifenemulsion auf der Haut und die entsprechende Seifenemulsion dafür
Flacon pour la distribution d'une émulsion de savon à barbe sur la peau et émulsion s'y rapportant

(30) Priority: 25.05.1989 IT 2064489
(43) Date of publication of application: 28.11.1990
(73) Proprietor: Baldan, Emilio, I-20122 Milan (IT)
(72) Inventor: Baldan, Emilio, I-20122 Milan (IT)
(74) Representative: Gervasi, Gemma, Dr.

(56) References cited:
- EP-A- 0 046 038
- US-A- 2 937 391
- US-A- 3 095 598
- US-A- 3 284 839
- US-A- 3 560 100

## Description

The traditional method of lathering before shaving is to use a cream prepared for this specific purpose, which is distributed with the aid of a brush and water.

This method has the drawback of being impractical in meeting the requirements of many situations of modern life such as during travelling for work reasons and during holidays.

To meet such requirements disposable razors have been invented, however these always additionally require a tube of shaving cream and a brush, which constitute an encumbrance and require their own case, into which the brush can be returned only when sufficiently dry.

US 3 284 839 relates to a roller-type applicator for shaving soap or similar cream. The shaving cream is fed to the roller for application by gently squeezing the container. The cream flows axially of the roller and so becomes distribute over the whole lenght of the roller. However, in this document a roller for applying a shaving cream is used.

Roll-on containers employing a spherical ball-like applicator are well-known to the art. The typical document showing this art includes US 3 560 100. This document on which the preamble of claim 1 is based, relates to a dispenser capable of being held in the hand and for applying creams or other viscous substances to the human body. However, this reference fails to suggest the use of the device for distributing a shaving emulsion over the skin.

US 2 937 391 relates to a ball-type dispenser for the application of thin films of perfumes, deodorants, medicaments, mucilage but not for dispensing a shaving soap emulsion.

NOVAK- " Die kosmetischen Präparate", 3 Auflage, Band 2, 1984, Verlag für chem. Industrie- H. Ziolkowsky K.G. Augsburg; chapter XXI.2 (page 777-779) relates to brushless O/W (oil in water) shaving cream. For this particular purpose a stearate cream is used , however this reference fails to suggest the use of roll-on pocket-sized bottle.

FEY-OTTE "Wörterbuch der Kosmetik", Wissenschaftliche Verlagsgesellschaft mbH Stuttgart-1985 relates in general to brushless shaving cream (page 220) mainly containing stearin, kaliumhydroxide and/or triethanolamine, glycerol or propylene glycol. No percentage of the different substances are provided.

We have now conceived a bottle for distributing a shaving soap emulsion, together with the soap emulsion itself, which obviate the drawbacks of the known art.

Said bottle containing the shaving soap emulsion is characterised by being provided in its upper portion with a ball which when applied to the skin rotates under the combined action of the pressure and movement exercised by the hand on the skin, to distribute the soap emulsion over the skin.

The soap emulsion is characterised by containing cetylstearyl alcohol, cetyltrimethylammonium chloride, citric acid, isopropyl alcohol, silicone emulsion, methylchloroisothiazolinone, methylisothiazolinone and other excipients of scenting type.

The characteristics and advantages of the pocket-size bottle for distributing a shaving soap emulsion over the skin and the relative soap emulsion according to the present invention will be more apparent from the following detailed description.

Said bottle is shown in the accompanying figures, in which:

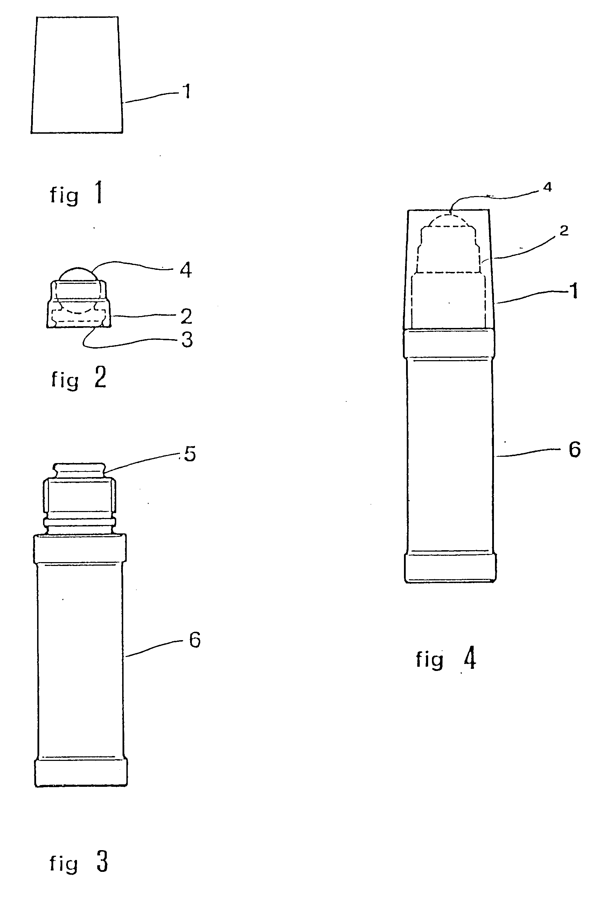

Figure 1 represents the bottle cap;
Figure 2 represents the element with the ball for distributing the soap suspension;
Figure 3 represents the container for the soap suspension;
Figure 4 represents the complete bottle ready for commercial distribution.

In the various figures the reference numeral 1 indicates the bottle cap, which is applied by screwing onto the upper portion of the container 6.

The reference numeral 2 indicates the element which supports the ball 4 and engages the recess 5 of the container 6 via the projection 3.

The cap 1 and container 6 are constructed of plastics material and preferably PVC. The element 2 is of plastics material, and preferably polyethylene The ball 4 is of plastics material, and preferably polypropylene.

The operation of the bottle according to the present invention is as follows: the soap emulsion is loaded into the container 6, the element 2 comprising the ball 4 is fitted to the top of the container 6, the ball 4 is brought into contact with the skin and under slight hand pressure is moved about while maintaining contact with the skin. During these movements under pressure, the ball rotates to entrain the soap suspension, which is distributed over the entire skin. After this distribution, shaving is carried out with a blade razor.

The emulsion according to the present invention comprises cetylstearyl alcohol, cetyltrimethylammonium chloride, citric acid, isopropyl alcohol, silicone emulsion, methylchloroisothi-azolinone and methylisothiazolinone. Said emulsion also comprises other excipients normally used in perfumery, such as scents, menthol, peppermint and possibly others.

The preferred composition of said emulsion, expressed in parts by weight, is as follows:

| | |
|---|---|
| Cetylstearyl alcohol | 1.35-2.70 |
| Cetyltrimethylammonium chloride | 0.80-2.00 |
| Citric acid | 0.15-0.30 |
| Scent | 0.50-1.00 |
| Isopropyl alcohol | 2.00-10.00 |
| Menthol crystals | 0.09-0.25 |
| Peppermint essential oil | 0.03-0.06 |
| Silicone emulsion | 1.00-2.00 |
| Methylchloroisothiazolinone | 0.00075 |
| Methylisothiazolinone | 0.00025 |
| Demineralized water | to make up to 100 |

Using this soap emulsion with the described bottle, we have found that rapid, uniform and complete distribution is obtained over the skin, which after this treatment is ready for shaving. A bottle containing 10 ml of emulsion is sufficient for four or five shaves.

The great advantage of using the bottle and soap suspension according to the present invention is apparent from the aforegoing description. In this respect, they replace the traditional brush and tube of shaving cream, with advantages in terms both of space saving and operation.

## Claims

1. A pocket-sized bottle provided at its open top end with a ball (4) which when applied to the skin rotates thereover under the combined action of pressure and movement imparted by hand and containing a soap emulsion, characterized in that the soap emulsion is a shaving soap emulsion and has the following composition :
| | |
|---|---|
| Cetylstearyl alcohol | 1.35-2.70 |
| Cetyltrimethylammonium chloride | 0.80-2.00 |
| Citric acid | 0.15-0.30 |
| Scent | 0.50-1.00 |
| Isopropyl alcohol | 2.00-10.00 |
| Menthol crystals | 0.09-0.25 |
| Peppermint essential oil | 0.03-0.06 |
| Silicone emulsion | 1.00-2.00 |
| Methylchloroisothiazolinone | 0.00075 |
| Methylisothiazolinone | 0.00025 |
| Demineralized water | to make up to 100 |

## Patentansprüche

1. Flasche in Taschengröße, die an ihrem offenen oberen Ende mit einer Kugel (4) versehen ist, die beim Aufbringen auf die Haut über diese unter der kombinierten Wirkung von Druck und Bewegung, die durch die Hand ausgeübt werden, rotiert und die Seifenemulsion enthält, dadurch **gekennzeichnet,** daß die Seifenemulsion eine Rasierseifenemulsion ist und die folgende Zusammensetzung hat:
| | |
|---|---|
| Cetylstearylalkohol | 1,35 - 2,70 |
| Cetyltrimethylammoniumchlorid | 0,80 - 2,00 |
| Zitronensäure | 0,15 - 0,30 |
| Geruchsstoff | 0,50 - 1,00 |
| Isopropylalkohol | 2,00 - 10,00 |
| Mentholkristalle | 0,09 - 0,25 |
| Pfefferminzöl | 0,03 - 0,06 |
| Siliconemulsion | 1,00 - 2,00 |
| Methylchlorisothiazolinon | 0,00075 |
| Methylisothiazolinon | 0,00025 |
| Entionisiertes Wasser | auf 100 |

## Revendications

1. Flacon de poche pourvu à son sommet ouvert d'une bille (4), qui, lorsqu'elle est appliquée sur la peau, roule à sa surface sous l'action combinée de la pression et du mouvement donné à la main, et contenant une émulsion de savon, caractérisé en ce que l'émulsion de savon est une émulsion de savon à barbe et possède la composition suivante :
| | |
|---|---|
| alcool cétylstéarylique | 1,35 - 2,70 |
| chlorure de cétyltriméthylammonium | 0,80 - 2,00 |
| acide citrique | 0,15 - 0,30 |
| parfum | 0,50 - 1,00 |
| alcool isopropylique | 2,00 - 10,00 |
| cristaux de menthol | 0,09 - 0,25 |
| huile essentielle de menthe poivrée | 0,03 - 0,06 |
| émulsion de silicone | 1,00 - 2,00 |
| méthylchloroisothiazolinone | 0,00075 |
| méthylisothiazolinone | 0,00025 |
| eau déminéralisée | q.s.p. compléter à 100 |
